# FASCICULE DE BREVET EUROPEEN

(11) **EP 1 019 112 B1**
(45) Date de publication et mention de la délivrance du brevet: **07.05.2003**
(21) Numéro de dépôt: 95931241.4
(22) Date de dépôt: 14.09.1995
(51) Int. Cl.: A61L 31/00

(54) **UTILISATION DE MEMBRANES COLLAGENIQUES COMME PROTHESES DE REGENERATION PERITONEALE**
VERWENDUNG VON KOLLAGENMEMBRANEN ALS PROTHESE FÜR PERITONEALE REGENERATION
USE OF COLLAGEN MEMBRANES AS PERITONEAL RENEWING PROSTHESES

(30) Priorité: 15.09.1994 FR 9411015; 30.06.1995 FR 9507908
(43) Date de publication de la demande: 19.07.2000
(73) Titulaire: SOFRADIM PRODUCTION, 01600 Trévoux (FR)
(72) Inventeur: ORLY, Isabelle, F-69001 Lyon (FR)
(74) Mandataire: Guerre, Dominique
(86) Numéro de dépôt international: FR9501177
(87) Numéro de publication internationale: WO96008277

(56) Documents cités:
- EP-A- 0 187 014
- WO-A-89/08467
- WO-A-93/02718

## Description

L'invention concerne essentiellement l'utilisation de gel de collagène pour la fabrication d'une plaque transparente, biocompatible, suturable ou agrafable, et résorbable, permettant la prévention des adhérences post-opératoires.

Une telle membrane peut être avantageusement utilisée en chirurgie pariétale, de préférence en chirurgie sous coelioscopie et en particulier pour remplacer temporairement le péritoine avant sa régénération dans les zones de dépéritonisation opératoire, et diminuer ainsi le risque d'adhérences pathogènes.

Dans le cadre de la description et des revendications, les termes "chirurgie pariétale" signifient toute chirurgie dans laquelle la paroi abdominale est concernée. D'autre part, dans le cadre de la description et des revendications, on entend par "plaque transparente" une membrane caractérisée en ce qu'il soit possible, dans les conditions d'utilisation, de voir suffisamment au travers pour être en mesure de l'agrafer ou de la suturer en évitant les zones défavorables telles que vaisseaux et nerfs.

L'une des complications les plus fréquentes, en chirurgie viscérale en particulier, est la formation d'adhérences consécutives à des résections péritonéales.

En effet, les zones de dépéritonisation opératoire, qu'elles concernent le péritoine pariétal ou le péritoine viscéral, et qui sont parfois des zones de taille importante, sont le siège d'une activité inflammatoire et/ou cicatricielle qui conduit à un accolement anormal entre deux surfaces ou segments d'organes normalement séparés.

Les complications liées aux adhérences post-opératoires peuvent être d'une gravité variable. Certaines sont très sérieuses. En chirurgie abdominale par exemple, les adhérences intrapéritonéales peuvent provoquer des occlusions intestinales. En gynécologie, les adhérences peuvent être cause de stérilité et sont parfois à l'origine de douleurs pelviennes, voire d'occlusions intestinales.

Un moyen d'éviter ou de diminuer le risque d'apparition de ce phénomène est d'intercaler, entre les tissus susceptibles de s'accoler, une plaque d'un produit résorbable, biocompatible, qui va séparer physiquement les deux tissus en question, pendant une durée nécessaire à la régénération du péritoine, puis qui sera résorbée en induisant une réaction inflammatoire la plus discrète possible.

Pour une utilisation en chirurgie coelioscopique, pratique de plus en plus courante compte tenu de ses nombreux avantages, la plaque évoquée ci-dessus doit de plus posséder des propriétés particulières liées à son mode d'introduction et de fixation dans l'organisme.

Ainsi, la plaque doit être aisément manipulable, c'est-à-dire d'une souplesse telle qu'elle puisse être roulée et passée dans un trocart d'environ 1 cm de diamètre, tout en étant en même temps suffisamment rigide et possédant une mémoire de forme telle qu'elle s'étale facilement une fois introduite dans la cavité abdominale.

Ensuite, elle doit pouvoir être fixée par sutures ou plus souvent par agrafes, ce qui suppose une résistance suffisante au déchirement, ceci au moment de la fixation et pendant la durée de temps nécessaire pour qu'elle soit réhabitée par les cellules de l'hôte.

Elle doit être également suffisamment résistante à la pression et à l'étirement pour pouvoir conserver son intégrité pendant le temps nécessaire à la reconstruction d'un nouveau péritoine, estimé à quelques jours.

Il apparaît que les matériaux actuellement connus pour leur utilisation dans le domaine de la chirurgie viscérale ne répondent pas de façon simultanée aux exigences énumérées ci-dessus.

Par ailleurs, les inventeurs se sont posé pour la première fois le nouveau problème technique de proposer une solution qui permette de discerner les vaisseaux sanguins de la zone sur laquelle la membrane est placée, de façon à pouvoir ainsi éviter ces vaisseaux lors de l'agrafage, ceci permettant d'éviter les hémorragies qui posent un problème tout à fait crucial en chirurgie coelioscopique, et en particulier dans les zones de dépéritonisation opératoire, c'est-à-dire des zones où le péritoine a été enlevé.

Les matériaux actuellement connus dans le domaine de la chirurgie coelioscopique ou viscérale ne sont pas adaptés.

Il est tout d'abord connu une plaque formée d'un treillis de copolymère d'acide lactique et glycolique enduit de collagène et commercialisé sous le nom de Treillis Vicryl Collagène® (Société Ethicon ®-Ethnor S.A. 92200 NEUILLY, France) mais qui n'est pas transparente et dont la résorption est lente, à savoir plusieurs semaines ou mois, ce qui peut être très défavorable en cas d'infections.

Par ailleurs, le brevet FR-94 06995 propose une plaque collagénique anti-adhérence non transparente et non adaptée pour une mise en place dans le cadre de la chirurgie coelioscopique, en particulier viscérale, du fait de ses propriétés adhésives.

Il est encore connu, par le Document FR-2.628.634 B1, un patch de chirurgie viscérale réalisé à partir d'un biomatériau formé de deux couches de collagène superposées et associées intimement. Il est indiqué que ce patch permet une bonne cicatrisation des viscères et qu'il est réalisé à partir d'un biomatériau qui se colle facilement. A la lecture des applications de ce patch, il apparaît qu'il n'est pas particulièrement destiné à la régénération du péritoine, ni surtout aux interventions sous coelioscopie. De plus, les présents inventeurs ont découvert que le patch décrit dans ce document présente une résistance mécanique relativement faible, en particulier une résistance faible au déchirement, ce qui engendrerait des risques de déchirure au niveau des sutures ou des agrafes et finalement un détachement de la plaque.

Les présents inventeurs ont également découvert que le patch selon ce dernier document serait d'un emploi inconfortable en chirurgie coelioscopique puisqu'il a la particularité de se coller facilement et qu'il est souple, deux aspects qui sont contraires à la nécessité pour le matériau de pouvoir s'étaler sans difficulté après avoir été roulé et introduit dans un trocart.

Ainsi, la présente invention a pour but principal de fournir un nouveau matériau formant membrane ou plaque de chirurgie, particulièrement pour la chirurgie pariétale, de préférence en chirurgie sous coelioscopie, en étant particulièrement adapté à la régénération du péritoine, qui soit en outre biocompatible, ayant une bonne résistance mécanique pour permettre une fixation par sutures ou agrafes, aisément manipulable dans les conditions de la chirurgie sous coelioscopie.

La présente invention a encore pour but de résoudre le nouveau problème technique de la fourniture d'un nouveau matériau formant membrane ou plaque de chirurgie, en particulier pour la chirurgie pariétale, de préférence en chirurgie sous coelioscopie, qui soit transparent afin de permettre au chirurgien de discerner les caractéristiques du site d'implantation de façon à pouvoir fixer la membrane ou plaque en évitant les zones défavorables telles que par exemple les vaisseaux sanguins, ceci permettant d'éviter les hémorragies qui posent un problème tout à fait crucial en chirurgie, et en particulier en chirurgie coelioscopique.

La présente invention a encore pour but de fournir un matériau qui évite la formation des adhérences post-opératoires et qui soit en outre particulièrement adapté pour permettre une régénération du péritoine.

La présente invention a encore pour but d'accélérer la régénération du péritoine.

Il a été découvert de façon tout à fait inattendue pour l'homme de l'art que l'utilisation de collagène, éventuellement réticulé par un agent de réticulation, du type décrit dans le document précédent du déposant WO 93/02718, permettait de résoudre complètement les problèmes techniques énoncés ci-dessus, de manière fiable et reproductible, utilisable à l'échelle industrielle et médicale, à un coût raisonnable.

Ainsi, la présente invention concerne une nouvelle utilisation d'un gel de collagène séché par un fluide gazeux non toxique tel que par exemple l'air ou l'azote pour la fabrication d'une membrane collagénique, éventuellement réticulée, transparente, biocompatible, suturable ou agrafable, et résorbable, et permettant la prévention des adhérences post-opératoires.

Selon le document WO 93/02718, l'utilisation de collagène concernait la régénération tissulaire guidée en parodontologie, par exemple pour réaliser le comblement de poches parondontales, ou le réhaussement des crêtes maxillo-mandibulaires, ou la régénération osseuse périimplantaire. Dans le cadre de cette utilisation antérieure, la membrane devait présenter une résorption lente et était de ce fait préparée à partir d'une membrane réticulée.

Il est également apparu que le patch décrit dans le document FR-2.628.634 B1 présente une résistance mécanique faible car il est constitué d'un film de collagène ou de gélatine obtenu par collage d'une solution sur du collagène fibreux, ce qui entraîne une redissolution partielle de ce collagène fibreux et qui résulte en un matériau présentant une résistance mécanique relativement faible.

Dans le cadre de la nouvelle utilisation objet de la présente invention, il est avantageux que la résorption soit relativement rapide, c'est-à-dire qu'elle ait lieu dans un délai inférieur à trois mois. En outre, les membranes de l'invention doivent permettre une régénération rapide du péritoine.

Ainsi, la présente invention concerne l'utilisation d'une membrane collagénique, biocompatible et résorbable, éventuellement réticulée par un agent de réticulation, pour l'obtention d'une plaque transparente, suffisamment résistante au déchirement pour être suturable ou agrafable, présentant une face anti-adhérente, à usage chirurgical pariétal, par exemple pour une régénération du péritoine ou une prévention des adhérences post-opératoires.

Préférentiellement, la membrane est susceptible d'être obtenue par séchage dudit gel de collagène dans un fluide gazeux non toxique, tel que par exemple l'air ou l'azote.

Par exemple, la membrane est susceptible d'être obtenue à partir d'un gel de collagène coagulé par un agent coagulant, ledit collagène coagulé étant ensuite soumis à un séchage dans un fluide gazeux non toxique, tel que par exemple l'air ou l'azote.

La membrane utilisée selon la présente invention peut avoir une structure mixte, susceptible d'être obtenue à partir d'une éponge de collagène sur laquelle a été coulé ledit gel de collagène.

La plaque obtenue selon la présente invention peut être utilisée en chirurgie sous coelioscopie, ou en chirurgie abdominale ou viscérale.

La présente invention concerne également une prothèse caractérisée par l'association solidaire d'un treillis synthétique, biocompatible, mécaniquement résistant pour être suturable ou agrafable, et d'au moins une membrane collagénique, disposée sur au moins une face dudit treillis, elle-même biocompatible et résorbable, anti-adhérente.

Le treillis synthétique peut être résorbable ou non.

La prothèse selon l'invention présente préférentiellement une face membrane collagénique et une face treillis.

La prothèse selon l'invention peut comprendre deux faces collagéniques de part et d'autre du treillis.

Préférentiellement, la membrane collagénique est continue.

A titre d'exemple, en combinaison, le maillage du treillis est tel qu'on puisse voir au travers, et la membrane collagénique est transparente, dans les conditions de son utilisation, par exemple après hydratation.

Préférentiellement, la membrane est réticulée, et présente éventuellement un taux de réticulation relativement faible du collagène. Par exemple, le taux de réticulation est tel qu'il apparaît une augmentation inférieure à 20°C, de préférence inférieure à 15°C, de la température de dénaturation du collagène, par rapport à la température de dénaturation du collagène natif de départ, non soumis à la réticulation.

La réticulation est susceptible d'être obtenue avec un agent de réticulation choisi parmi le groupe consistant en une aldéhyde, en particulier la glutaraldéhyde, et le diphénylphosphorylazide ou un carbodiimide, de préférence le diphénylphosphorylazide ou un carbodiimide.

A titre d'exemple, la membrane présente une épaisseur comprise entre 0,05 millimètre et 1 millimètre, de préférence entre 0,1 millimètre et 0,5 millimètre.

Préférentiellement, la prothèse selon l'invention est susceptible d'être obtenue à partir d'un treillis synthétique, biocompatible, mécaniquement résistant pour être suturable ou agrafable, par l'un quelconque des procédés suivants :
- coulage d'un gel de collagène ou d'atélocollagène, éventuellement mélangé à un glycosaminoglycanne, sur ledit treillis synthétique ;
- coulage dans un récipient dudit gel, sur lequel est déposé ledit treillis, l'étape de coulage étant suivie d'une étape de séchage, dans un fluide gazeux non toxique, par exemple l'air ou l'azote.

Toute prothèse selon l'invention peut alors être utilisée pour les réfections de la paroi abdominale, permettant d'associer l'avantage de la membrane collagénique quant à sa propriété de régénération du péritoine et de prévention des adhérences et celui du treillis synthétique quant à sa résistance mécanique temporaire ou non. Cette prothèse pourra donc être utilisée pour les cures d'éventration et de hernie.

L'éponge de collagène précitée peut être obtenue de manière classique comme décrit par exemple dans le document précédent WO 93/02718, par lyophilisation d'un gel de collagène. Pour préparer l'éponge, on peut aussi utiliser un mélange de collagène ou atélocollagène et de glycosaminoglycanne. Ce mélange peut aussi être neutralisé avant d'être lyophilisé pour obtenir ladite éponge. Selon une autre caractéristique avantageuse, l'éponge peut être comprimée sous pression, en particulier sous une pression d'au moins 150 bars, avant de procéder au coulage du gel de collagène et à son séchage.

Le procédé de réticulation précité est bien connu à l'homme de l'art. Pour un procédé de réticulation par le DPPA, on peut se reporter à une autre demande antérieure publiée sous le No. FR-A-2.645.870 = US-A-5,264,551.

Comme collagène, on peut utiliser du collagène natif, en particulier de type I ou de type III.

Selon une variante de réalisation particulière, on peut aussi utiliser de l'atélocollagène, bien que cela soit moins préféré.

Selon un autre mode de réalisation particulier, on utilise un mélange d'un gel de collagène ou atélocollagène et de glycosaminoglycanne, ce mélange étant éventuellement réticulé par l'agent de réticulation après son séchage.

Dans le cadre de l'invention, la membrane obtenue est transparente. Celle-ci reste transparente dans de larges limites d'épaisseur. De préférence, l'épaisseur de la membrane est comprise entre 0,05 millimètre (50 micromètres) et 1 millimètre, de préférence entre 0,1 millimètre et 0,5 millimètre (500 micromètres), la limite inférieure étant préférée en raison du fait que cette épaisseur minimale permet d'obtenir une résistance mécanique suffisante pour être agrafée ou suturée sans déchirement. L'épaisseur maximale est préférée pour maintenir la membrane transparente, ainsi que pour permettre à la membrane d'être roulée sans difficulté, introduite dans un trocart et déroulée encore sans difficulté sur la zone de fixation.

Dans le cadre de l'invention, on peut utiliser comme glycosaminoglycanne un glycosaminoglycanne de structure, en particulier l'acide hyaluronique, le chondroïtine-4-sulfate, le çhondroïtine-6-sulfate, le dermatane-sulfate, l'héparane-sulfate, le kératane-sulfate, l'héparine et ses dérivés, en particulier les héparines de bas poids moléculaire compris entre environ 2 000 et environ 10 000.

La proportion relative de glycosaminoglycanne relativement au collagène ou à l'atélocollagène est de préférence comprise entre 18 et 25% en poids.

Le mélange du glycosaminoglycanne avec le collagène ou l'atélocollagène a lieu sous forme de solution. Par exemple, le glycosaminoglycanne est sous forme d'une solution aqueuse de glycosaminoglycanne contenant de 0,5 à 4% en poids, plus particulièrement de 0,5 à 2% en poids, et encore de préférence environ 1% en poids de glycosaminoglycanne. De même, le collagène ou l'atélocollagène peut être sous forme d'une solution aqueuse ayant une concentration entre 0,3 et 2% en poids, et encore de préférence entre 0,4 et 0,8% en poids de collagène ou d'atélocollagène. Cette solution de collagène ou d'atélocollagène présente en pratique l'aspect d'un gel. La solution de collagène ou d'atélocollagène peut être préparée selon l'invention par dissolution de fibres de collagène ou d'atélocollagène dans une solution aqueuse légèrement acide. En particulier, il peut s'agir d'une solution aqueuse d'acide acétique 0,1M.

On peut prévoir d'amener le mélange de collagène, ou d'atélocollagène, et de glycosaminoglycanne à un pH voisin de la neutralité et en particulier à un pH compris entre 6,5 et 8. On peut utiliser à cet effet une solution aqueuse d'hydroxyde de sodium.

Selon une autre variante de réalisation avantageuse de l'invention, on peut utiliser un gel de collagène à l'état coagulé par suite d'une coagulation d'un gel de collagène par un agent coagulant comme décrit dans la demande précédente WO93/02718. Dans ce cadre, on réalise la coagulation du gel par un agent coagulant comprenant une solution ammoniacale ayant de préférence un effet déshydratant. Une telle solution ammoniacale déshydratante est une solution ammoniacale organique utilisant de l'acétone comme agent déshydratant. Il se produit un effet de synergie par la combinaison acétone/ammoniaque pour la coagulation du gel et pour l'élimination de l'eau présente dans le gel.

On préfère avantageusement une proportion relative acétone/ammoniaque comprise entre 50/50 et 80/20 en volume, une proportion volumique encore particulièrement préférée étant de 70/30 d'acétone/ammoniaque.

Dans le cas où les quantités de gel à coaguler sont relativement importantes, on réalise un renouvellement de la solution coagulante en cours de coagulation.

Selon une variante de réalisation particulière, le gel peut être coulé à travers une filière de section et de forme appropriées pour obtenir un gel coagulé de forme appropriée. Lorsque la section de la filière est rectangulaire, on obtient ainsi un film qui, par la suite, va constituer la membrane par séchage.

Le gel coagulé obtenu peut ensuite être soumis à une réticulation par l'agent de réticulation précité en réglant le degré de réticulation de préférence pour permettre une résorption relativement rapide, comme précédemment décrit.

Selon encore une autre variante avantageuse de l'invention, on peut utiliser un gel de collagène ou d'atélocollagène, éventuellement mélangé à un glycosaminoglycanne, coulé sur le treillis synthétique précité, résorbable ou non, ou coulé dans un récipient et sur lequel sera déposé ledit treillis et/ou éventuellement une deuxième couche de gel pourra être coulée sur le treillis.

Ainsi, selon que ledit gel de collagène ou d'atélocollagène est déposé sur une seule ou sur les deux face du treillis, on obtient respectivement un produit présentant une face collagène et une face treillis ou deux faces collagène de part et d'autre du treillis selon le système du sandwich.

La présente invention permet, un procédé de traitement chirurgical selon lequel on introduit par voie chirurgicale, de préférence sous coelioscopie, dans le corps d'un patient ayant besoin de ladite chirurgie, une membrane de chirurgie transparente, biocompatible, suturable ou agrafable, à résorption relativement rapide, permettant la prévention des adhérences post-opératoires, préparée par séchage dans un fluide gazeux non toxique tel que par exemple l'air ou l'azote d'un gel de collagène, en disposant une face de la membrane contre la paroi, au niveau de la zone où l'on veut régénérer le péritoine et prévenir la formation d'adhérences, en prenant soin d'éviter les zones défavorables tels que vaisseaux et nerfs, en jouant sur la transparence de la membrane, puis on agrafe ladite membrane sur la paroi à l'aide d'une pince pour agrafage, de préférence adaptée pour un agrafage sous coelioscopie; en permettant ainsi une régénération rapide du péritoine et la prévention des adhérences post-opératoires.

Selon une variante de réalisation avantageuse du procédé de traitement, la membrane est introduite dans la cavité abdominale selon une technique classique de chirurgie sous coelioscopie par l'intermédiaire d'un trocart dans lequel la membrane est enroulée sur elle même après avoir été préalablement stérilisée, par exemple par irradiation γ, puis hydratée par trempage dans une solution physiologiquement compatible pendant quelques secondes. Cette solution est de préférence une solution de sérum physiologique.

Selon une autre variante du procédé de traitement, ce procédé est caractérisé en ce que l'on utilise une prothèse constituée d'une membrane collagénique associée à un treillis synthétique sur une seule ou sur ses deux faces, notamment dans les cures de hernies ou d'éventrations.

Avantageusement, lorsque la prothèse présente une face membrane collagénique et une face treillis, cette prothèse est étendue de façon à ce que la membrane collagénique s'interpose entre les plans susceptibles d'être le siège d'adhérences post-opératoires indésirables.

Selon une autre variante du procédé de traitement, celui-ci est caractérisé en ce que la prothèse est implantée en site intrapéritonéal, en la disposant contre la paroi abdominale, face collagénique en regard des viscères.

D'autres caractéristiques du procédé de traitement apparaîtront également clairement à l'homme de l'art à partir de la description précédente et de la description suivante qui va être réalisée, comme cela est apparent à l'homme de l'art.

Dans le cadre de l'un quelconque des aspects de l'invention, il est à noter que le séchage dans un fluide gazeux non toxique, tel que par exemple l'air ou l'azote, est de préférence réalisé à la température ambiante. Ce séchage peut être réalisé par passage d'un flux du fluide gazeux non toxique laminaire stérile. Cependant, ce flux du fluide gazeux peut être éventuellement chauffé à une température non supérieure à la température de dénaturation du collagène, qui est bien connue et aisément déterminable par l'homme de l'art.

On comprend ainsi que l'invention permet d'aboutir à tous les avantages techniques déterminants précédemment énoncés ainsi qu'aux avantages techniques qui apparaîtront à l'homme de l'art à partir de la description explicative suivante de l'invention faite en référence à trois modes de réalisation actuellement préférés de l'invention, donnés simplement à titre d'illustration et qui ne sauraient donc en aucune façon limiter la portée de l'invention. Dans les Exemples, les pourcentages sont donnés en poids, sauf indication contraire.

### Exemple I :

### Préparation d'une membrane de collagène simple

### A - Extraction du collagène natif et préparation du gel

Un gel est préparé à partir de peaux de veaux préalablement lavées et épilées par un mélange de chaux-sulfure (chaux : 4%, sulfure de sodium : 3%).

Elle est ensuite déchaulée dans un bain contenant du chlorure d'ammonium (2%) et du métabisulfite de sodium (0,5%). Elle est ensuite neutralisée, puis les sels sont éliminés par deux lavages à l'eau. Elle est alors broyée, puis lavée par du tampon phosphate pH 7,8 (dihydrogénophosphate de potassium 0,78 g/l et monohydrogénophosphate disodique 21,7 g/l). Le phosphate est ensuite éliminé par deux lavages successifs à l'eau permutée.

Le broyat est alors acidifié par une solution d'acide acétique à 10%, la quantité d'acide acétique étant de 5% par rapport au collagène. Le broyat est alors malaxé afin d'obtenir une pâte homogène. Cette pâte est ensuite diluée pour obtenir un gel ayant une concentration de 0,75% en collagène natif.

### B - Préparation du film

Le gel obtenu est dégazé sous vide puis coulé dans un bain coagulant à travers une filière rectangulaire dont la section a une hauteur de 0,3 mm. La solution coagulante est un mélange acétone/ammoniaque (V/V 70/30) qui est renouvelé tous les 250 ml de gel.

Le film obtenu est alors séché à l'air à température ambiante sur un support plastique en polytétrafluoroéthylène. Une fois sec, le film est aisément décollé de son support.

Le film est alors placé dans une solution de glycérol à 10%. Il est ensuite séché à l'air et stérilisé par radiation γ à une dose de 25 kGy (kiloGray). La membrane obtenue est ainsi transparente et aisément agrafable ou suturable.

### Exemple II

### Préparation d'une membrane mixte de collagène glycosaminoglycanne réticulé

### A - Extraction du collagène natif et préparation du gel

Un gel est préparé à partir de peaux de veaux préalablement lavées et épilées par un mélange de chaux-sulfure (chaux : 4%, sulfure de sodium : 3%).

Elle est ensuite déchaulée dans un bain contenant du chlorure d'ammonium (2%) et du métabisulfite de sodium (0,5%). Elle est ensuite neutralisée, puis les sels sont éliminés par deux lavages à l'eau. Elle est alors broyée, puis lavée par du tampon phosphate pH 7,8 (dihydrogénophosphate de potassium 0,78 g/l et monohydrogénophosphate disodique 21,7 g/l). Le phosphate est ensuite éliminé par deux lavages successifs à l'eau permutée.

Le broyat est alors acidifié par une solution d'acide acétique à 10%, la quantité d'acide acétique étant de 5% par rapport au collagène. Le broyat est alors malaxé afin d'obtenir une pâte homogène. Cette pâte est ensuite diluée pour obtenir un gel ayant une concentration de 0,75% en collagène natif.

### B - Préparation du chondroïtine-4-sulfate

Des cloisons nasales d'agneau débarrassées des tissus musculaires et adipeux sont hachées et broyées par extrusion à travers une grille comportant des trous de 4 mm ; le broyat est placé pendant 24 h à une température de 6°C dans une tampon de chlorure de potassium (11,8 g/l de KCL, 78,8 mg/l de cystéine, ETDA 180 mg/l) renfermant 1% de papaïne "MERCK". La proportion étant de 130 g de broyat pour 1 de tampon.

Le surnageant est séparé du culot par centrifugation en continu à l'aide d'une décanteuse tournant à 400 tr/min. Au surnageant, sont alors ajoutés 40 g/l d'acide trichloracétique. Le précipité est éliminé par centrifugation en continu selon la technique précédente. Le surnageant est neutralisé à l'aide de soude en pastilles. Le mélange est alors dialysé contre de l'eau désionisée et stérile à l'aide de boyaux dont le seuil de coupure est compris entre 6000 et 8000 daltons. La solution dialysée est lyophilisée. Le chondroïtine-4-sulfate est obtenu à l'état sec.

### C - Préparation de l'éponge collagène chondroïtine-4-sulfate

A 1 l de gel à 0,75% de collagène, est ajouté 1,87 g de chondroïtine-4-sulfate. Après neutralisation, le mélange est agité puis lyophilisé. L'éponge obtenue est pressée pendant 15 s sous une pression de 150 bars.

### D - Préparation de la membrane mixte

Le gel de collagène à 1% est coulé sur l'éponge comprimée à l'aide d'une filière dont la section a une hauteur de 0,3 cm. 10 ml de gel sont déposés sur 35 cm² d'éponge. La membrane ainsi réalisée est séchée à l'air libre.

### E - Réticulation chimique de la membrane

La membrane séchée est incubée pendant 24 h à 4°C dans une solution de DMF contenant du DPPA à raison de 5 microlitres dans 25 millilitres de DMF pour 1 g de produit sec. Le DPPA est éliminé de la membrane par rinçage dans une solution de tampon borate pH 8,9 (tétraborate de sodium 0,04M , acide borique 0,04M) puis rincée 5 fois à l'eau désionisée avant d'être placée dans une solution aqueuse de glycérol à 10%.

La membrane est ensuite séchée à l'air et stérilisée par radiation γ à une dose de 25 kGy (kiloGray).

Les membranes selon l'invention ainsi préparées sont transparentes, agrafables ou suturables. Elles peuvent être utilisées de préférence en une épaisseur comprise entre 0,1 millimètre et 1 millimètre comme membranes ou plaques de chirurgie, de préférence de chirurgie sous coelioscopie, en particulier de chirurgie pariétale.

Dans le cadre de l'invention, les membranes peuvent être fabriquées sous forme de plaques ayant diverses tailles. Généralement, la dimension des membranes ou plaques sera comprise, pour les applications envisagées, entre 3 x 3 et 30 x 30 cm. Toutefois, il est encore possible et préférable que la plaque soit recoupée au moment de son utilisation, en fonction de la taille de la zone dépéritonisée.

Il est à noter que les plaques fabriquées selon cet Exemple II présentent les propriétés mécaniques suivantes avant et après réticulation, ces plaques présentant une épaisseur de 0,2 mm environ :

Avant réticulation :
- résistance à la rupture : 0,22 ± 0,10 daN
- résistance à la déchirure : 0,11 ± 0,04 daN

Après réticulation :
- résistance à la rupture : 0,49 ± 0,08 daN
- résistance à la déchirure : 0,26 ± 0,01 daN.

Il est à noter que, dans le cas de la préparation d'une membrane mixte, il est préféré de réaliser une réticulation car cette réticulation permet de renforcer la cohésion entre la partie éponge, préparée selon la partie C de l'Exemple II, et la partie film, préparée selon la partie D de l'Exemple II.

Ainsi, cette réticulation a pour effet d'améliorer la résistance mécanique. La réticulation permet aussi de régler la durée de résorption.

On peut par exemple utiliser une membrane de forme carrée ayant une dimension de 15 x 15 cm par exemple pour former un cylindre susceptible de passer dans un trocart de 1 cm de diamètre et de s'étaler facilement une fois introduite dans la cavité abdominale.

Ces membranes ou plaques ont été utilisées en chirurgie conformément à l'Exemple ci-après.

### Exemple III

### Utilisation des membranes selon l'invention en chirurgie sous coelioscopie pour la régénération du péritoine Expérimentation animale in vivo chez le porc

Des membranes sous forme de plaques carrées de 5 x 5 cm de côté telles qu'obtenues aux Exemples I et II ci-dessus, c'est-à-dire à l'état sec, stérilisées par radiations γ, sont tout d'abord hydratées par trempage dans une solution de sérum physiologique pendant quelques secondes, puis elles sont enroulées sur elles-mêmes de façon à pouvoir les faire passer dans un trocart d'1 cm de diamètre environ. Elles sont introduites dans la cavité abdominale selon une technique classique de chirurgie sous coelioscopie par l'intermédiaire de ce trocart de 1 cm de diamètre. Puis elles sont étalées aisément à l'aide d'instruments classiquement utilisés en chirurgie coelioscopique. Les plaques sont placées contre la paroi abdominale, après dépéritonisation préalable de la zone destinée à reproduire une situation pathologique que les membranes selon l'invention permettent de traiter. Les plaques adhèrent spontanément sur les zones où elles ont été déposées. En outre, étant donné qu'elles sont transparentes, l'agrafage en est particulièrement aisé. Les membranes ou plaques sont agrafées avec des agrafes en titane à l'aide d'une pince pour agrafage sous coelioscopie. Les zones-témoins ont été dépéritonisées mais n'ont pas reçu de membrane, à titre comparatif.

L'expérimentation a été réalisée sur trois porcs, chacun ayant reçu plusieurs membranes sous forme de plaques carrées de 5 x 5 cm ci-dessus décrites. Plusieurs zones-témoins ont également été réalisées dans le péritoine.

Les animaux sont sacrifiés après des délais de 1 semaine, 5 semaines et 3 mois.

Des contrôles coelioscopiques ont été effectués dans ces mêmes délais, avant le sacrifice, de façon à pouvoir juger du comportement des membranes ou plaques en situation. Ces contrôles ainsi que les examens effectués à l'autopsie ont montré :
- L'absence d'adhérences,
- La régénération rapide du péritoine puisque, dès le premier délai (1 semaine), les membranes sont déjà recouvertes par une fine pellicule de tissu.
- Après 5 semaines, la résorption des plaques est très avancée, les adhérences sont toujours absentes, l'implant est apparemment complètement recouvert par un péritoine néo-formé.
- Après trois mois, les zones implantées sont difficiles à repérer. Les membranes ou plaques ont totalement disparu ou ne persistent qu'à l'état de traces, le péritoine présent sur le site d'implantation présentant un aspect normal.
- Il est à remarquer qu'il n'a pas été noté de différences majeures entre les plaques obtenues à l'Exemple I et celles obtenues à l'Exemple II, dans les conditions de l'essai.
- En revanche, dans les zones-témoins, les zones dépéritonisées qui n'ont pas été recouvertes d'une membrane ou plaque sont le siège d'adhérences qui persistent pendant toute la durée de l'expérimentation.

### Exemple IV :

### Préparation d'une membrane de collagène séché à l'air associée à un treillis synthétique

### A - Extraction du collagène natif et préparation du gel

Ces phases sont réalisées conformément à celles décrites au paragraphe A des exemples I et II ci-dessus.

### B - Préparation de la membrane collagénique associée au treillis

Le gel obtenu est dégazé sous vide, puis une couche de gel d'environ 0,5 cm d'épaisseur est déposée sur un treillis synthétique transparent déposé dans un récipient, ledit treillis synthétique biocompatible étant disponible dans le commerce étant réalisé en fibres de polymère synthétique ayant un diamètre de 100 µ définissant ainsi des pores de diamètre 263 µ environ.

Après séchage à l'air, on obtient une membrane continue de collagène d'environ 200 micromètres d'épaisseur à la surface du treillis synthétique.

Le matériau obtenu est stérilisé par radiation γ à une dose de 25 kGy. La membrane obtenue est ainsi transparente et aisément suturable ou agrafable.

### Exemple V

### Utilisation des membranes selon l'invention associées à un treillis synthétique pour la régénération du péritoine dans les cas de réfection de paroi en chirurgie sous coelioscopie. Expérimentation animale in vivo chez le porc

Des membranes associées à un treillis synthétique sous forme de plaques carrées de 5 x 5 cm de côté telles qu'obtenues à l'exemple IV ci-dessus ainsi que des plaques de treillis synthétiques identiques à celui de l'Exemple IV seules sont implantées sous coelioscopie chez le porc selon le protocole expérimental décrit à l'exemple III ci-dessus.

Les plaques formées par l'association de membranes collagéniques associées à un treillis synthétique sont implantéees face collagénique en regard des viscères.

Les contrôles coelioscopiques effectués avant le sacrifice ainsi que les examens effectués à l'autopsie ont montré que :

### a - Après 1 semaine d'implantation :

- la membrane collagénique constitue une zone de clivage permettant la libération des éventuelles adhérences post-opératoires précoces qui peuvent se former ;
- l'absence de membrane collagénique (treillis seul) se traduit par la formation d'adhérences beaucoup plus difficiles à libérer.

### b - Après 5 et 12 semaines d'implantation :

- la membrane collagénique associée au treillis s'est résorbée, sa résorption s'étant accompagnée de la libération spontanée des éventuelles adhérences qui avaient été observées au délai antérieur et d'une parfaite repéritonisation du site implanté ;
- certains sites ayant reçu le treillis seul sont encore le siège, au moins partiellement, d'adhérences persistantes.

Ainsi, l'invention permet bien de favoriser la régénération du péritoine et la prévention des adhérences post-opératoires.

Ainsi, l'invention permet bien de résoudre l'ensemble des problèmes techniques précédemment énoncés, en fournissant une nouvelle membrane transparente collagénique pour la chirurgie sous coelioscopie, aisément agrafable ou suturable et particulièrement utile dans la chirurgie viscérale, et pariétale, en particulier pour une régénération rapide du péritoine et la prévention des adhérences post-opératoires.

L'invention comprend naturellement tous les moyens constituant des équivalents techniques des moyens décrits ainsi que leurs diverses combinaisons.

## Revendications

1. Prothèse **caractérisée par** l'association solidaire d'un treillis synthétique, biocompatible, mécaniquement résistant pour être suturable ou agrafable, et d'au moins une membrane collagénique, disposée sur au moins une face dudit treillis, elle-même biocompatible et résorbable, anti-adhérente.

2. Prothèse selon la revendication 1, **caractérisée en ce que** le treillis synthétique est résorbable ou non.

3. Prothèse selon la revendication 1, **caractérisée en ce qu'**elle présente une face membrane collagénique et une face treillis.

4. Prothèse selon la revendication 1, **caractérisée en ce qu'**elle comprend deux faces collagéniques de part et d'autre du treillis.

5. Prothèse selon la revendication 1, **caractérisée en ce que** la membrane collagénique est continue.

6. Prothèse selon la revendication 1, **caractérisée en ce que**, en combinaison, le maillage du treillis est tel qu'on puisse voir au travers, et la membrane collagénique est transparente, dans les conditions de son utilisation, par exemple après hydratation.

7. Prothèse selon la revendication 1, **caractérisée en ce que** la membrane est réticulée, et présente éventuellement un taux de réticulation relativement faible du collagène.

8. Prothèse selon la revendication 7, **caractérisée en ce que** le taux de réticulation est tel qu'il apparaît une augmentation inférieure à 20° C, de préférence inférieure à 15° C, de la température de dénaturation du collagène, par rapport à la température de dénaturation du collagène natif de départ, non soumis à la réticulation.

9. Prothèse selon la revendication 7, **caractérisée en ce que** la réticulation est susceptible d'être obtenue avec un agent de réticulation choisi parmi le groupe consistant en une aldéhyde, en particulier la glutaraldéhyde, et le diphénylphosphorylazide ou un carbodiimide, de préférence le diphénylphosphorylazide ou un carbodiimide.

10. Prothèse selon la revendication 1, **caractérisé en ce que** la membrane présente une épaisseur comprise entre 0,05 millimètre et 1 millimètre, de préférence entre 0,1 millimètre et 0,5 millimètre.

11. Prothèse, **caractérisée en ce qu'**elle est susceptible d'être obtenue à partir d'un treillis synthétique, biocompatible, mécaniquement résistant pour être suturable ou agrafable, par l'un quelconque des procédés suivants :
- coulage d'un gel de collagène ou d'atélocollagène, éventuellement mélangé à un glycosaminoglycanne, sur ledit treillis synthétique ;
- coulage dans un récipient dudit gel, sur lequel est déposé ledit treillis, l'étape de coulage étant suivie d'une étape de séchage, dans un fluide gazeux non toxique, par exemple l'air ou l'azote.

12. Utilisation d'une membrane collagénique, biocompatible et résorbable, éventuellement réticulée par un agent de réticulation, pour l'obtention d'une plaque transparente, suffisamment résistante au déchirement pour être suturable ou agrafable, présentant une face anti-adhérente, à usage chirurgical pariétal, par exemple pour une régénération du péritoine ou une prévention des adhérences post-opératoires

13. Utilisation selon la revendication 12, **caractérisé en ce que** la membrane est susceptible d'être obtenue par séchage dudit gel de collagène dans un fluide gazeux non toxique, tel que par exemple l'air ou l'azote.

14. Utilisation selon la revendication 12, **caractérisée en ce que** la membrane est susceptible d'être obtenue à partir d'un gel de collagène coagulé par un agent coagulant, ledit collagène coagulé étant ensuite soumis à un séchage dans un fluide gazeux non toxique, tel que par exemple l'air ou l'azote.

15. Utilisation selon la revendication 12, **caractérisée en ce que** la membrane a une structure mixte susceptible d'être obtenue à partir d'une éponge de collagène sur laquelle a été coulé ledit gel de collagène.

16. Utilisation selon la revendication 12, pour la chirurgie sous coelioscopie

17. Utilisation selon la revendication 12, pour la chirurgie abdominale ou viscérale

## Patentansprüche

1. Prothese, **gekennzeichnet durch** die feste Zusammenfügung eines synthetischen, biokompatiblen, mechanisch widerstandsfähigen Gitterwerkes, das nähbar oder klammerbar ist, und zumindest eine auf zumindest einer Seite des Gitterwerks angeordneten Collagenmembran, die selbst biokompatibel und resorbierbar, jedoch antihaftend ist.

2. Prothese nach Anspruch 1, **dadurch gekennzeichnet, dass** das synthetische Gitterwerk resorbierbar ist oder nicht.

3. Prothese nach Anspruch 1, **dadurch gekennzeichnet, dass** diese eine Collagenmembranseite und eine Gitterwerkseite aufweist.

4. Prothese nach Anspruch 1, **dadurch gekennzeichnet, dass** diese zwei Collagenseiten beidseits des Gitterwerks aufweist.

5. Prothese nach Anspruch 1, **dadurch gekennzeichnet, dass** die Collagenmembran kontinuierlich ist.

6. Prothese nach Anspruch 1, **dadurch gekennzeichnet, dass**, in Kombination, das Maschenwerk des Gitterwerkes derart ist, dass man durch es hindurch sehen kann, und dass die Collagenmembran durchsichtig ist, und zwar unter den Bedingungen seiner Anwendung, d.h. beispielsweise nach einer Hydratisierung.

7. Prothese nach Anspruch 1, **dadurch gekennzeichnet, dass** die Membran vernetzt ist, und ggf. einen relativ schwachen Vernetzungsgrad des Collagens aufweist.

8. Prothese nach Anspruch 7, **dadurch gekennzeichnet, dass** der Vernetzungsgrad derart ist, dass dieser eine Verbesserung der Denaturationstemperatur des Collagens unterhalb bis 20°C, vorzugsweise unterhalb bis 15°C zeigt, und zwar im Vergleich zu der Denaturierungstemperatur des natürlichen ausgänglichen Collagens, das keiner Vernetzung unterworfen wurde.

9. Prothese nach Anspruch 7, **dadurch gekennzeichnet, dass** die Vernetzung mit einem Vernetzungsagens, ausgewählt aus der Gruppe bestehend aus einem Aldehyd, insbesondere dem Glutaraldehyd, und Diphenylphosphorylazid oder einem Carbodiimid, vorzugsweise Diphenylphosphorylazid oder einem Carbodiimid, erhältlich ist.

10. Prothese nach Anspruch 1, **dadurch gekennzeichnet, dass** die Membran eine Dicke von etwa 0,05 mm bis 1 mm, vorzugsweise von 0,1 mm bis 0,5 mm, aufweist.

11. Prothese, **dadurch gekennzeichnet, dass** diese aus einem synthetischen, biokompatiblen, mechanisch widerstandfähigen Gitterwerk erhältlich ist, das nähbar oder klammerbar ist, und zwar nach einem der folgenden Verfahren, nämlich
- Gießen eines Collagen- oder eines Atelocollagen-Gels, ggf. gemischt mit einem Glycosaminoglycan, auf das synthetische Gitterwerk;
- Gießen des Gels in ein Behältnis, auf das das Gitterwerk gebracht ist, wobei der Gießschritt von einem Trockenschritt in einem nichttoxischen, gasförmigen Fluidum, beispielsweise Luft oder Stickstoff, gefolgt ist.

12. Verwendung einer biokompatiblen und resorbierbaren, ggf. durch ein Vernetzungsagens vernetzten Collagenmembran zum Herstellen einer transparenten Platte, die ausreichend widerstandsfähig und reißfest ist, um nähbar oder klammerbar zu sein, welche eine antihaftende Seite aufweist zur parietalen chirurgischen Verwendung, beispielsweise zu der Regeneration des Bauchfells oder zur Prävention von postoperativen Anhaftungen.

13. Verwendung nach Anspruch 12, **dadurch gekennzeichnet, dass** die Membran durch Trocknen des Collagengels in einem nichttoxischen gasförmigen Fluidum wie beispielsweise Luft oder Stickstoff erhältlich ist.

14. Verwendung nach Anspruch 12,**dadurch gekennzeichnet, dass** die Membran aus einem durch ein Koagulierungsagens koaguliertem Collagengel erhältlich ist, wobei das koagulierte Collagen anschließend einer Tracknung in einem nichttoxischen gasförmigen Fluid wie beispielsweise Luft oder Stickstoff unterworfen wird.

15. Verwendung nach Anspruch 12, **dadurch gekennzeichnet, dass** die Membran eine gemischte Struktur aufweist, die aus einem Collagenschwamm erhältlich ist, auf den das Kollgengel gegossen wurde.

16. Verwendung nach Anspruch 12 für die laparoskopische Chirurgie.

17. Verwendung nach Anspruch 12 für die abdominale oder viszerale Chirurgie.

## Claims

1. Prosthesis, **characterized by** the interlinked combination of a synthetic, biocompatible lattice, which is mechanically resistant so that it can be sutured or stapled, and of at least one collagen membrane, placed on at least one face of said lattice, itself biocompatible and resorbable, and which is anti-adhesive.

2. Prosthesis according to Claim 1, **characterized in that** the synthetic lattice may or may not be resorbable.

3. Prosthesis according to Claim 1, **characterized in that** it has a collagen membrane face and a lattice face.

4. Prosthesis according to Claim 1, **characterized in that** it comprises two collagen faces on either side of the lattice.

5. Prosthesis according to Claim 1, **characterized in that** the collagen membrane is continuous.

6. Prosthesis according to Claim 1, **characterized in that**, in combination, the meshing of the lattice is such that it is possible to see through, and the collagen membrane is transparent, under the conditions for its use, for example after hydration.

7. Prosthesis according to Claim 1, **characterized in that** the membrane is crosslinked, and optionally exhibits a relatively low degree of crosslinking of the collagen.

8. Prosthesis according to Claim 7, **characterized in that** the degree of crosslinking is such that an increase of less than 20°C, preferably less than 15°C, in the temperature of denaturation of the collagen appears, relative to the temperature of denaturation of the starting native collagen, not subjected to the crosslinking.

9. Prosthesis according to Claim 7, **characterized in that** the crosslinking can be obtained with a crosslinking agent chosen from the group consisting of an aldehyde, in particular glutaraldehyde, and diphenylphosphorylazide or a carbodiimide, preferably diphenylphosphorylazide or a carbodiimide.

10. Prosthesis according to Claim 1, **characterized in that** the membrane is between 0.05 millimetre and 1 millimetre, preferably between 0.1 millimetre and 0.5 millimetre, thick.

11. Prosthesis, **characterized in that** it can be obtained from a synthetic, biocompatible lattice which is mechanically resistant so that it can be sutured or stapled, using any one of the following methods:
- pouring a gel of collagen or of atelocollagen, optionally mixed with a glycosaminoglycan, onto said synthetic lattice;
- pouring said gel into a container, onto which gel is placed said lattice, the pouring step being followed by a step of drying, in a gaseous nontoxic fluid, for example air or nitrogen.

12. Use of a biocompatible, resorbable collagen membrane, optionally crosslinked with a crosslinking agent, for producing a transparent sheet sufficiently resistant to tearing so that it can be sutured or stapled, exhibiting an anti-adhesive face, for use in parietal surgery, for example for regeneration of the peritoneum or prevention of postoperative adhesions.

13. Use according to Claim 12, **characterized in that** the membrane can be obtained by drying said collagen gel in a gaseous nontoxic fluid, such as, for example, air or nitrogen.

14. Use according to Claim 12, **characterized in that** the membrane can be obtained from a collagen gel coagulated with a coagulant, said coagulated collagen then being subjected to drying in a gaseous nontoxic fluid, such as, for example, air or nitrogen.

15. Use according to Claim 12, **characterized in that** the membrane has a mixed structure which can be obtained from a collagen sponge onto which said collagen has been poured.

16. Use according to Claim 12, for surgery by coelioscopy.

17. Use according to Claim 12, for abdominal or visceral surgery.
